# EUROPÄISCHE PATENTANMELDUNG

(11) **EP 2 384 694 A1**
(43) Veröffentlichungstag der Anmeldung: **09.11.2011**
(21) Anmeldenummer: 10162064.9
(22) Anmeldetag: 05.05.2010
(51) Int. Cl.: A61B 5/00, B31D 1/00

(54) **Verfahren zur Herstellung eines Membranrings oder Teststreifenrings**

(71) Anmelder: Roche Diagnostics GmbH, 68305 Mannheim (DE); F. Hoffmann-La Roche AG, 4070 Basel (CH)
(72) Erfinder: Harttig, Herbert, Dr., 67434, Neustadt (DE)
(74) Vertreter: Pfiz, Thomas

(57) **Zusammenfassung**

Die Erfindung betrifft ein Verfahren zur Herstellung eines Membranrings oder Teststreifenrings für eine diagnostische Testeinrichtung bei dem
a) ein langgestreckter Streifen (26) durch quer zur Streifenlängsrichtung verlaufende Einschnitte (24) in Segmente (20) unterteilt wird,
b) wobei die Einschnitte (24) nur bis zu einer Restbreite des Streifens (26) geführt werden, so dass zwischen den an die Einschnitte (24) angrenzenden Segmenten (20) eine Materialbrücke (38) erhalten bleibt;
c) der Streifen (26) unter Zusammenführen seiner Enden zu einem Ring (22) geschlossen wird, wobei die zu den Materialbrücken (38) hin verlaufenden Schnittkanten der Einschnitte (24) jeweils einen spitzen Winkel (α) einschließen;
d) der Ring (22) als Membranring oder Teststreifenring für die Testeinrichtung in einer Tragstruktur (12) eingesetzt wird.

## Beschreibung

Die Erfindung betrifft ein Verfahren zur Herstellung eines Membranrings oder Teststreifenrings für eine diagnostische Testeinrichtung insbesondere zur Untersuchung von Körperflüssigkeiten. Die Erfindung betrifft weiter ein Ringmagazin als Verfahrensprodukt für eine diagnostische Testeinrichtung.

Im Bereich der medizinischen Diagnostik ist der Einsatz von Mikrofiltrationsmembranen in Form von Streifen oder Scheiben an sich bekannt. Auch die Verwendung von scheibenförmigen Magazinen in Handgeräten für Blutzuckermessungen ist beispielsweise aus der W02009/037192 bekannt. Nachteilig beim direkten Zuschnitt von Flachmaterialringen ist der Verlust an Material, der besonders bei schmalen Ringen sehr ausgeprägt ist. So werden bei einem Ring von 49 mm Außendurchmesser und 39 mm Innendurchmesser von einer quadratischen Ausgangsfläche, aus der der Ring ausgeschnitten wird, nur 28,8% genutzt. Werden die Ringe in dichtest möglicher Anordnung aus einer Ausgangsfläche geschnitten, so können theoretisch maximal 33,2 % genutzt werden, praktisch weniger. Bei den hohen Kosten von Membranen spielt die Ausbeute an nutzbarer Fläche vor allem für Massenprodukte eine wichtige ökonomische Rolle. Zudem ist die Handhabung von einzelnen Membranflächen zeitlich und mechanisch aufwändig und fehleranfällig.

Ausgehend hiervon liegt der Erfindung die Aufgabe zugrunde, die im Stand der Technik bekannten Verfahren und Vorrichtungen weiter zu verbessern und im Bereich diagnostischer Anwendungen den Materialeinsatz und die Handhabung von Verbrauchsmitteln zu optimieren.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, für eine Mehrzahl von Einzeltests in einer ringförmigen Anordnung jeweils ein analytisches Materialsegment insbesondere als Magazin bereitzustellen. Dementsprechend wird in verfahrensmäßiger Hinsicht vorgeschlagen, dass
a) ein vorzugsweise durch Zuschneiden eines flächigen oder bandförmigen Ausgangsmaterials bereitgestellter langgestreckter Streifen durch quer (d.h. rechtwinklig oder schräg) zur Streifenlängsrichtung verlaufende Einschnitte in Segmente unterteilt wird,
b) wobei die Einschnitte nur bis zu einer ggf. variierenden Restbreite des Streifens geführt werden, so dass zwischen den an die Einschnitte angrenzenden Segmenten eine Materialbrücke erhalten bleibt;
c) der Streifen unter Zusammenführen seiner Enden zu einem Ring geschlossen wird, wobei die zu den Materialbrücken hin verlaufenden Schnittkanten der Einschnitte jeweils einen spitzen Winkel einschließen;
d) der Ring als Membranring oder Teststreifenring für die Testeinrichtung in einer Tragstruktur eingesetzt wird.

Auf diese Weise kann das direkte Ausstanzen eines Materialrings vermieden und das zu verwerfende Material während des Herstellungsprozesses minimiert werden. Außerdem kann die Automatisierung durch eine lineare Vorfertigung beispielsweise von Rolle zu Rolle vereinfacht werden. Zugleich wird durch die Segmentierung eine Aufteilung in Einzeltests besonders vereinfacht.

Gemäß einer bevorzugten Ausgestaltung sollte der Streifen bis auf eine als Materialbrücke verbleibende Restbreite von 0,1 bis 1,0 mm eingeschnitten werden, um eine hinreichende Biegsamkeit zu gewährleisten. Bevorzugt wird eine einheitliche Restbreite gewählt.

Eine vorteilhafte Ausführung sieht vor, dass der Streifen einheitlich von einem seiner Längsränder her eingeschnitten wird, so dass die Materialbrücken an dem anderen Längsrand verbleiben. Liegen die Materialbrücken am Außenrand des nachfolgend gebildeten Rings, ist die Überlappung der Segmente maximal, während bei Materialbrücken am Innenrand keine Überlappung erfolgt.

Gemäß einer weiteren vorteilhaften Variante wird der Streifen auf jeweils einer Linie von seinen beiden Längsrändern her unter Erhaltung einer Materialbrücke beidseitig eingeschnitten.

Die Einschnitte lassen sich vorteilhafterweise mit einem Schneid- oder Stanzwerkzeug oder durch Laserschneiden erzeugen. Der Schnitt mit einem Laser verdichtet die Schnittkante und stabilisiert dadurch die Materialbrücke. Daher wird ein Laserschnitt bevorzugt.

Herstellungstechnisch ist es von Vorteil, wenn der eingeschnittene Streifen vor der Ringbildung in seiner Längsrichtung tordiert wird, so dass die Segmente bezüglich einer durch die Materialbrücken verlaufenden Kippachse mit fortlaufend zunehmendem Winkel gekippt sind. Dadurch ist eine nachfolgende Ringformung möglich, ohne dass die Schnittkanten aneinander verhaken.

Vorteilhafterweise werden die Segmente bei der Ringbildung gemäß Schritt c) sukzessive auf einer ebenen Ringfläche abgelegt und dabei aus einer Kippstellung in eine gegenseitige Überlappstellung gebracht.

In dem vorgenannten Verfahrensschritt c) wird der Ring vorteilhafterweise so geformt, dass die Materialbrücken eine Kreislinie aufspannen, wobei die Enden des Streifens sich auf der Kreislinie kontaktieren.

Eine weitere Verbesserung des Verfahrensproduktes lässt sich dadurch erzielen, dass die Segmente bei der Ringbildung paarweise überlappend aufeinander abgelegt werden, wobei die durch Schnittkanten der Einschnitte begrenzten Überlappungen von der Ringinnenseite zu der jeweiligen Materialbrücke hin verlaufen.

Um den gebildeten Ring zu stabilisieren, ist es vorteilhaft, wenn benachbarte Segmente vorzugsweise unter Einwirkung von Wärme und/oder Druck überlappend miteinander verbunden, insbesondere verschweißt oder verklebt werden. Günstig ist es auch, wenn bei einer gegenseitigen Verbindung der Segmente zugleich eine Fixierung des Rings auf der Tragstruktur, bevorzugt in einem Magazin erfolgt.

Ein besonders bevorzugter Einsatz sieht ein Ausgangsmaterial vor, das durch eine mit einer reaktiven Testchemie beschichtete Folie oder durch eine für die Körperflüssigkeit zumindest partiell durchlässige Membran gebildet wird. Vorteilhaft kann ein Membranring auf einen Teststreifenring unter paarweisem Kontakt der beiderseitigen Segmente aufgebracht werden. Denkbar ist es auch, dass einzelne Testfelder auf die Segmente eines Membranrings aufgebracht werden, oder umgekehrt.

In Zusammenhang mit diagnostischen Anwendungen ist es von Vorteil, wenn die Segmente als Verbrauchsmittel in einem Ringmagazin bevorzugt in Kammern getrennt für jeweils einen Einzeltest bereitgestellt werden. Bevorzugt werden solche Kammern mit jeweils einem Stech- bzw. Nadelelement zur Gewinnung von Körperflüssigkeit bestückt.

Gegenstand der Erfindung ist auch ein Ringmagazin für eine diagnostische Testeinrichtung insbesondere zur Untersuchung von Körperflüssigkeiten mit einer Tragstruktur und einem darin eingesetzten Membranring oder Teststreifenring hergestellt nach dem erfindungsgemäßen Verfahren, wobei die Segmente ein Filter oder ein Nachweiselement für jeweils einen Einzeltest bilden. Ein solcher Membranring oder Teststreifenring weist demnach einen Streifen auf, der durch Randeinschnitte quer zur Streifenlängsrichtung in Segmente unterteilt ist, wobei die Einschnitte nur bis zu einer ggf. variierenden Restbreite des Streifens verlaufen, so dass zwischen den an die Einschnitte angrenzenden Segmenten eine Materialbrücke erhalten bleibt, und wobei der Streifen zu einem Ring geschlossen ist, so dass die zu den Materialbrücken hin verlaufenden Schnittkanten der Einschnitte jeweils einen spitzen Winkel einschließen.

Bei einem solchen Ringmagazin kann die Tragstruktur Öffnungen bzw. Fenster für den Transfer von Körperflüssigkeit aufweisen, wobei die Öffnungen von jeweils einem Segment des Membranrings oder Teststreifenrings abgedeckt sind. Bevorzugt weist die Tragstruktur ringförmig angeordnete Testkammern auf, die vorteilhafterweise jeweils ein Stechelement enthalten, und die jeweils einem Segment des Membran- oder Teststreifenrings für die Durchführung eines Einzeltests zugeordnet sind.

Im Folgenden wird die Erfindung anhand eines in der Zeichnung schematisch dargestellten Ausführungsbeispiels näher erläutert. Es zeigen
- Fig. 1: ein Ringmagazin für ein diagnostisches Testgerät mit einem nur hälftig gezeigten Teststreifenring in perspektivischer Darstellung;
- Fig. 2: eine schaubildliche Darstellung einer Vorrichtung zur Vorfertigung eines eingeschnittenen Teststreifens;
- Fig. 3: verschiedene Stufen einer materialsparenden Bildung des Teststreifenrings aus dem vorgefertigten Teststreifen in abgebrochener Draufsicht.

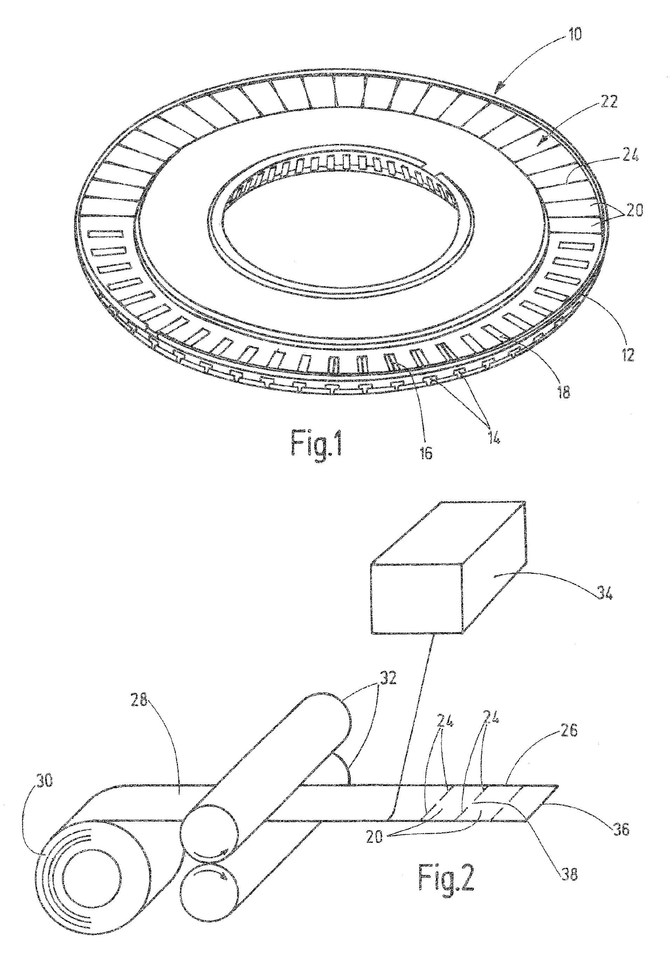

Das in Fig. 1 dargestellte Ringmagazin lässt sich als Verbrauchsmaterial in ein nicht gezeigtes mobiles Analysegerät für Blutzuckeranalyen einsetzen. Es umfasst zu diesem Zweck ein scheibenförmiges Gehäuse 12 als Tragstruktur, in welchem eine Vielzahl von kreisförmig angeordneten Kammern 14 zur Aufnahme von radial ausschiebbaren Stechelementen 16 enthalten sind, wobei auf dem Gehäusedeckel über den Kammern 12 jeweils eine Öffnung 18 vorgesehen ist, über die bei einem Körpereinstich gewonnenes Blut für einen photometrischen Glukosenachweis auf ein Segment 20 eines Teststreifenrings 22 übertragen werden kann. Denkbar ist es auch, dass ein Membranring als Filter zum Blutübertrag zwischen den Stechelementen 16 und einem Testring angebracht wird.

Um einen solchen Teststreifen- bzw. Membranring 22 möglichst materialsparend herstellen zu können, ist eine Ringfertigung aus einem durch Einschnitte 24 segmentierten Streifenmaterial vorgesehen, wie es nachstehend näher erläutert wird. Damit kann das direkte Ausstanzen eines Flachmaterialrings vermieden und das zu verwerfende Material während des Herstellungsprozesses minimiert werden.

Fig. 2 veranschaulicht die Vorfertigung eines eingeschnittenen Bandstreifens 26. Dabei wird ein Band 28 als Ausgangsmaterial von einer Vorratsspule 30 abgezogen und über Transportwalzen 32 geführt. Ein Laser 34 als Schneidgerät ermöglicht mittels Laserstrahl 36 das Ablängen des Streifens 26 an Streifenenden 36 und das Einbringen der Einschnitte 24 unter Bildung der Segmente 20 in dem Bandmaterial. Alternativ ist es auch denkbar, von einem großflächigen Ausgangsmaterial zunächst Streifen abzuschneiden und diese dann mit den Quereinschnitten zu versehen.

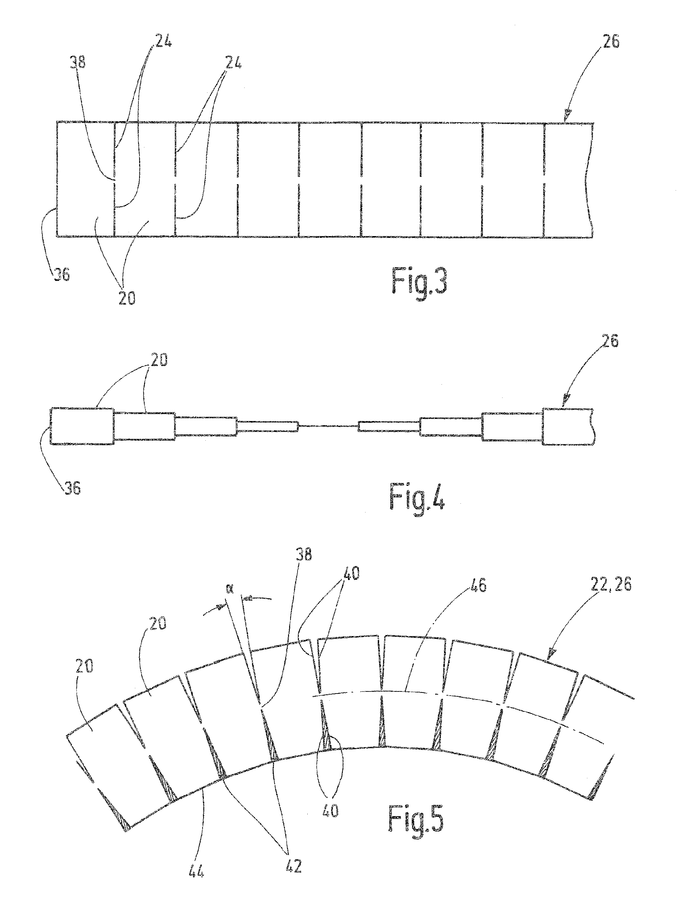

Wie auch aus Fig. 3 ersichtlich, ist bei dem gezeigten Ausführungsbeispiel der Streifen 26 auf jeweils einer Querlinie rechtwinklig zu den Längsrändern beidseitig eingeschnitten, so dass eine zentrale Materialbrücke 38 zwischen benachbarten Segmenten 20 erhalten bleibt. Möglich ist es auch, dass der Streifen einheitlich von einem Längs- bzw. Seitenrand her rechtwinklig eingeschnitten wird, so dass die Materialbrücken dann an dem gegenüberliegenden anderen Längsrand angeordnet sind. Zweckmäßig liegen die Einschnitte 24 in Längsrichtung des Streifens 26 gesehen in gleichem Abstand voneinander, um deckungsgleiche Segmente 20 zu erhalten.

Beispielsweise kann ein Bandmaterial von 90 µm Dicke in einen Streifen mit einer Länge von 140 mm und einer Breite von 5 mm geschnitten werden, während die Einschnitte im Längsabstand von 2,8 mm liegen und von beiden Längsrändern aus jeweils 2,4 mm weit quer verlaufen, so dass eine Materialbrücke von 0,2 mm verbleibt.

Wie in Fig. 4 gezeigt, wird der eingeschnittene Streifen 26 vor der Ringbildung in seiner Längsrichtung tordiert, so dass die Segmente 20 bezüglich einer durch die Materialbrücken 38 verlaufenden Längsachse fortlaufend mit zunehmendem Winkel gekippt bzw. verdreht sind und entsprechend in der Draufsicht unterschiedlich breit erscheinen. Durch diese Maßnahme wird eine kollisionsfreie Ringbildung mit gegenseitigem Überlapp der Segmente 20 ermöglicht.

Fig. 5 illustriert die Ausbildung des Ringes 22 aus dem vorgefertigten Streifen 26 gemäß Fig. 3 und 4. Dabei werden die Segmente 20 sukzessive auf einer ebenen Ringfläche, zweckmäßig direkt auf dem Gehäusedeckel abgelegt. Beim Ablegen wird die Kippstellung der Segmente 20 in eine gegenseitige Überlappstellung zurückgeführt. Die durch die einander gegenüberliegenden Schnittkanten 40 der Einschnitte 24 begrenzten Überlappbereiche 42 verlaufen dann von der Ringinnenseite 44 zu der jeweiligen Materialbrücke 38 hin, während die radial äußeren Schnittkanten 40 auf unter einem spitzen Winkel α auseinanderklaffen. In dem so gebildeten Ring 26 spannen die Materialbrücken eine Kreislinie 46 auf, wobei die beiden Streifenenden 36 sich auf der Kreislinie kontaktieren.

Zweckmäßig werden die Segmente 20 in den Überlappbereichen 42 durch Einwirkung von Wärme und Druck miteinander verschweißt, beispielsweise mittels eines entsprechend strukturierten Heizstempels oder mittels eines transparenten Stempels und Laserlicht. Dabei kann zugleich eine stoffschlüssige Fixierung auf dem Gehäusedeckel erreicht werden.

## Patentansprüche

1. Verfahren zur Herstellung eines Membranrings oder Teststreifenrings für eine diagnostische Testeinrichtung insbesondere zur Untersuchung von Körperflüssigkeiten bei dem
a) ein vorzugsweise durch Zuschneiden eines flächigen oder bandförmigen Ausgangsmaterials bereitgestellter langgestreckter Streifen (26) durch quer zur Streifenlängsrichtung verlaufende Einschnitte (24) in Segmente (20) unterteilt wird,
b) wobei die Einschnitte (24) nur bis zu einer Restbreite des Streifens (26) geführt werden, so dass zwischen den an die Einschnitte (24) angrenzenden Segmenten (20) eine Materialbrücke (38) erhalten bleibt;
c) der Streifen (26) unter Zusammenführen seiner Enden zu einem Ring (22) geschlossen wird, wobei die zu den Materialbrücken (38) hin verlaufenden Schnittkanten der Einschnitte (24) jeweils einen spitzen Winkel (α) einschließen;
d) der Ring (22) als Membranring oder Teststreifenring für die Testeinrichtung in einer Tragstruktur (12) eingesetzt wird.

2. Verfahren nach Anspruch 1, **dadurch gekennzeichnet, dass** der Streifen (26) bis auf eine als Materialbrücke (38) verbleibende Restbreite von 0,1 bis 1,0 mm eingeschnitten wird.

3. Verfahren nach Anspruch 1 oder 2, **dadurch gekennzeichnet, dass** der Streifen (26) einheitlich von einem seiner Längsränder her eingeschnitten wird, so dass die Materialbrücken (38) an dem anderen Längsrand verbleiben.

4. Verfahren nach Anspruch 1 oder 2, **dadurch gekennzeichnet, dass** der Streifen (26) auf jeweils einer Linie von seinen beiden Längsrändern her unter Erhaltung einer Materialbrücke (38) beidseitig eingeschnitten wird.

5. Verfahren nach einem der Ansprüche 1 bis 4, **dadurch gekennzeichnet, dass** die Einschnitte (24) mit einem Schneid- oder Stanzwerkzeug oder durch Laserschneiden erzeugt werden.

6. Verfahren nach einem der Ansprüche 1 bis 5, **dadurch gekennzeichnet, dass** der eingeschnittene Streifen (26) vor der Ringbildung in seiner Längsrichtung tordiert wird, so dass die Segmente (20) bezüglich einer durch die Materialbrücken (38) verlaufenden Kippachse mit fortlaufend zunehmendem Winkel gekippt sind.

7. Verfahren nach einem der Ansprüche 1 bis 6, **dadurch gekennzeichnet, dass** die Segmente (20) sukzessive auf einer ebenen Ringfläche abgelegt und dabei aus einer Kippstellung in eine gegenseitige Überlappstellung gebracht werden.

8. Verfahren nach einem der Ansprüche 1 bis 7, **dadurch gekennzeichnet, dass** der Ring (22) derart geformt wird, dass die Materialbrücken (38) eine Kreislinie (46) aufspannen, wobei die Enden des Streifens (26) sich auf der Kreislinie kontaktieren.

9. Verfahren nach einem der Ansprüche 1 bis 8, **dadurch gekennzeichnet, dass** die Segmente (20) bei der Ringbildung paarweise überlappend aufeinander abgelegt werden, wobei die durch Schnittkanten der Einschnitte (24) begrenzten Überlappungen (42) von der Ringinnenseite zu der jeweiligen Materialbrücke (38) hin verlaufen.

10. Verfahren nach einem der Ansprüche 1 bis 9, **dadurch gekennzeichnet, dass** benachbarte Segmente (20) vorzugsweise unter Einwirkung von Wärme und/oder Druck überlappend miteinander verbunden, insbesondere verschweißt oder verklebt werden.

11. Verfahren nach einem der Ansprüche 1 bis 10, **dadurch gekennzeichnet, dass** bei einer gegenseitigen Verbindung der Segmente (20) zugleich eine Fixierung des Rings (22) auf der Tragstruktur (12) erfolgt.

12. Verfahren nach einem der Ansprüche 1 bis 11, dagekennzeichnet, dass das Ausgangsmaterial durch eine mit einer reaktiven Testchemie beschichtete Folie oder durch eine für die Körperflüssigkeit zumindest partiell durchlässige Membran gebildet wird.

13. Verfahren nach einem der Ansprüche 1 bis 12, **dadurch gekennzeichnet, dass** die Segmente (20) als Verbrauchsmittel in einem Ringmagazin (10) für jeweils einen Einzeltest bereitgestellt werden.

14. Ringmagazin für eine diagnostische Testeinrichtung insbesondere zur Untersuchung von Körperflüssigkeiten mit einer Tragstruktur (12) und einem darin eingesetzten Membranring oder Teststreifenring (22) hergestellt nach einem der vorhergehenden Ansprüche, wobei die Segmente (20) ein Filter oder ein Nachweiselement für jeweils einen Einzeltest bilden.

15. Ringmagazin nach Anspruch 14, **dadurch gekennzeichnet, dass** die Tragstruktur (12) eine Mehrzahl von Öffnungen (18) für den Transfer von Körperflüssigkeit aufweist, und dass die Öffnungen (18) von jeweils einem Segment (20) des Membranrings oder Teststreifenrings (22) abgedeckt sind.
